(19) Europäisches Patentamt
European Patent Office
Office européen des brevets

(11) Numéro de publication : **0 162 877 B1**

(12) **FASCICULE DE BREVET EUROPÉEN**

(45) Date de publication du fascicule du brevet : **04.05.88**

(51) Int. Cl.⁴ : **C 07 K 5/06**, C 12 Q 1/00// C07D263/44, C07C129/12, C07K5/08

(21) Numéro de dépôt : **84904142.1**

(22) Date de dépôt : **22.11.84**

(86) Numéro de dépôt international : **PCT/FR 84/00275**

(87) Numéro de publication internationale : **WO/8502404 (06.06.85 Gazette 85/13)**

(54) **Méthode de dosage d'enzymes.**

(30) Priorité : **24.11.83 FR 8318973**

(43) Date de publication de la demande : **04.12.85 Bulletin 85/49**

(45) Mention de la délivrance du brevet : **04.05.88 Bulletin 88/18**

(84) Etats contractants désignés : **AT BE CH DE FR GB LI LU NL SE**

(56) Documents cités :
**DE-A- 2 944 086**
**US-A- 3 455 784**
**TETRAHEDRON LETTERS, No. 27, 1965, Pergamon Press Ltd., Oxford (GB), H. Brockmann et al.: "Zur Cyclisierung steroisomerer Hexapeptide", pages 2291-2295, see page 2292, formula VI**
**COLLECTION OF CZECHOSLOVAK CHEMICAL COMMUNICATIONS, Vol. 29, No. 11, 1964, Prague(CS), Y. Chen-Su et al.: "Amino acids and peptides. XLIV. Synthesis of three diastereomeric cyclohexapeptides containing glycine, phenylalanine and leucine", pages 2633-2647, see page 2634, formula Alld; page 2635, formula B11d, page 2641, last two paragraphs**
**COLLECTION OF CZECHOSLOVAK CHEMICAL COMMUNICATIONS, Vol. 32, No. 11, 1967, Prague (CS), L. Mladenova-Orlinova et al.: "Amino acids and peptides, LXXVII. Further diastereomeric cyclohexapeptides containing glycine, phenylalanine and leucine; configurational conditions for the presence of cis**

(73) Titulaire : **FLORK, Michel Louis André**
**60, rue de Bellevue**
**F-63400 Chamalières (FR)**

**BOUTHILLON, Jean Claude Maurice Joseph**
**9, square Saint Charles**
**F-75012 Paris (FR)**

**BOUTHILLON, Marie-France épouse SARAVANE**
**Dourdé par Esplens**
**F-47600 Nerac (FR)**

(72) Inventeur : **FLORK, Michel Louis André**
**60, rue de Bellevue**
**F-63400 Chamalières (FR)**
Inventeur : **BOUTHILLON, Jean Claude Maurice Joseph**
**9, square Saint Charles**
**F-75012 Paris (FR)**
Inventeur : **BOUTHILLON, Marie-France épouse SARAVANE**
**Dourdé par Esplens**
**F-47600 Nerac (FR)**

(74) Mandataire : **Chanet, Jacques**
**Conseil en Brevets 129 Avenue de Royat B.P. 27**
**F-63400 Chamalières (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



Jouve, 18, rue St-Denis, 75001 Paris, France

peptide bonds", pages 4070-4081, see page 4073, formule EIId; page 4079, paragraph 4
CHEMICAL ABSTRACTS, Vol. 87, No. 3, 18 July 1977, Columbus, Ohio (US), A.M. Islam et al.: "Synthesis of some N-phthalyl peptides and cyclohexapeptides", see page 698, 699,abstract 23722x
CHEMICAL ABSTRACTS, Vol. 97, No. 15, 11 October 1982, Columbus, Ohio (US), M.A. Coletti-Previero et al. "Amino acid hydroxamates as inhibitors of the human enkephalin-degrading aminopeptidase", see page 288, abstract 122785h
Chemical Abstracts, Vol. 97, formula Index, July-December 1982, Columbus, Ohio (US), see page 427f, formula C7H15N503
Le dossier contient des informations techniques présentées postérieurement au dépôt de la demande et ne figurant pas dans le présent fascicule.

**Description**

La présente invention a pour objet une méthode de dosage d'enzymes en mettant en œuvre des dérivés de peptides plus particulièrement ceux de ces dérivés dont le carboxyle terminal est engagé dans une fonction hydrazide ou hydroxamate, de tels dérivés étant notamment destinés au dosage, par voie enzymatique, des paramètres sanguins.

On connaît par une publication DE-A-2 944 086 une méthode de dosage de l'activité spécifique des enzymes, par exemple de certains facteurs sanguins, au moyen de réactifs peptidiques comportant une terminaison 4-méthyle-coumaryl-7-amide ; la méthode procède de la libération de la terminaison et de son dosage par fluorescence.

Cependant le dosage par fluorescence exige un milieu homogène et transparent, ce qui n'est pas le cas du sang entier, alors que la méthode de l'invention permet d'opérer directement sur le sang entier ; en outre les dosages par fluorescence sont réputés moins sensibles que les dosages par voie électrochimique.

Selon la présente invention les dérivés sus-visés ont pour formule générale (I) :

$$Z_1 - Y - (Z_2)\,NHZ_3 \qquad (I)$$

dans laquelle :

$Z_1$ représente notamment un reste d'aminoacide choisi parmi les radicaux D-valyl, D-propyl, D-phénylalanyl, L-isoleucyl, L-glutamyl, L-pyroglutamyl, (N-benzoyl-L-isoleucyl-L-glutamyl) et N-(carbométhoxypropionyl-L-arginyl) ou de peptides ;

Y est un reste d'amino-acide choisi plus particulièrement dans le groupe constitué par un radical L-leucyl, L-phénylalanyl, L-pipécolyl, glycyl et L-propyl ou tout autre amino-acide ; et,

$Z_2$ est un reste d'amino-acide choisi plus particulièrement dans le groupe constitué par un reste L-lysyl, L-arginyl, L-tyrosyl, et L-valyl ou tout autre amino-acide ; et,

$Z_3$ représente un radical de formule $NH_2$, $NHR_1$, ou OH, $OR_1$ (dans laquelle $R_1$ est un radical alcoyle inférieur, un radical alcoyle inférieur substitué, un radical aryle ou un radical aryle substitué).

L'invention concerne plus précisément les composés de formule générale ($I_A$)

$$Z'_1 - Y' - (L - arginyl)\,NH - Z_3 \qquad (I_A)$$

dans laquelle :

$Z'_1$ est un radical aminé choisi dans le groupe constitué par le radical D-propyl, D-valyl, D-phénylalanyl (N-benzoyl L-isoleucyl-L-glutamyl), D-isoleucyl et L-glutamyl ;

Y' est un radical aminé choisi dans le groupe constitué par un radical L-phénylalanyl, L-leucyl, L-pipécolyl, glycyl, et L-prolyl ;

$Z_3$ est défini comme précédemment.

L'invention concerne également des dérivés plus simples de formule générale (II)

$$X_1 - arginyl - (NH)\,Z_3 \qquad (II)$$

dans laquelle :

$X_1$ est le reste acyle carboxylique, aliphatique ou aromatique, ayant de 1 à 10 atomes de carbone ; et

$Z_3$ est défini comme précédemment.

Parmi les composés de formule générale (I) ou de formule générale (II), on retiendra plus particulièrement :

— les hydroxamates de formule générale ($I_B$)

$$Z_1 - Y - Z_2\,NHOH \qquad (I_B)$$

dans laquelle les radicaux $Z_1$, Y et $Z_2$ sont définis comme précédemment ;

— les hydrazides non substitués de formule générale ($I_C$)

$$Z_1 - Y - Z_2\,NH - NH_2 \qquad (I_C)$$

dans laquelle les radicaux Y, $Z_1$ et $Z_2$ sont définis comme précédemment ; et,

— les hydrazides substitués de formule générale

$$Z_1 - Y - Z_2\,NH - NH - R_2 \qquad (I_D)$$

dans laquelle :

$Z_1$, $Z_2$ et Y sont définis comme précédemment ;

$R_2$ est un radical alcoyle inférieur ayant de 1 à 6 atomes de carbone en chaîne droite ou ramifiée, un

radical alcoyle inférieur substitué par un ou plusieurs hydroxyles, alcoxyles ou acyloxy, un radical aryle mono ou bicyclique substitué par un à trois substituants choisis parmi les radicaux halo, alcoyle inférieur, alcoxyle inférieur, trifluorométhyle, trifluorométhoxy, trifluorométhylthio, cyano, aminosulfonyle ou cycloalcoyle.

Parmi ceux-ci, on citera plus particulièrement à titre de composés plus spécialement utiles :

— la D-valyl-L-leucyl-L-lysyl hydrazide,
— la D-prolyl-L-phénylalanyl-L-arginyl hydrazide,
— la D-valyl-L-leucyl-L-arginyl-hydrazide,
— le D-valyl-L-leucyl-L-arginyl-hydroxamate,
— la D-phénylalanyl-L-pipécolyl-L-arginyl-hydrazide,
— la N-benzoyl-L-isoleucyl-L-glutamyl-glycyl-L-arginyl-hydrazide,
— la N-carbométhoxypropionyl-L-arginyl-L-propyl-L-tyrosyl-hydrazide,
— la L-pyroglutamyl-L-prolyl-L-valyl-méthyl-hydrazide,
— la L-pyroglutamyl-L-prolyl-L-valyl-isobutyl-hydrazide,
— le D-isoleucyl-L-prolyl-L-arginyl-hydroxamate,
— le glutamyl-glycyl-L-arginyl-hydroxamate,
— le N-formyl-L-arginyl-hydroxamate,
— la N-formyl-L-arginyl-N-méthyl-hydrazide,
— la N-formyl-L-arginyl-N-hydroxy-éthylhydrazide,
— la N-benzoyl-L-arginyl-hydrazide.

L'invention a également pour objet un procédé de préparation des composés de formule générale (I) qui se caractérise par le fait que l'on forme le N carboxy-anhydride de l'acide aminé Y par l'action du phosgène à température voisine de 50 °C et condense le N carboxyanhydride en milieu alcalin aqueux avec l'amino-acide $Z_2H$ pour former après acidification le peptide de formule générale (III)

$$Y - Z_2 \qquad \text{(III)}$$

et que l'on renouvelle cette opération en formant le N carboxyanhydride de l'amino-acide $Z_1$ et en le couplant par le même procédé sur le peptide $Y - Z_2$ pour obtenir après acidification le nouveau peptide de formule générale IV

$$Z_1 - Y - Z_2 \qquad \text{(IV)}$$

dans laquelle $Z_1$, $Z_2$ et Y sont définis comme précédemment, puisque l'on soumet celui-ci à l'estérification à l'aide d'un sulfate dialcoylique en présence d'un alcanol pour obtenir un alcoylsulfate d'ester de dipeptide, sépare celui-ci, le convertit en base d'ester d'amino-acide par alcalinisation à l'aide d'un hydroxyde ou un carbonate de métal alcalin puis le soumet à l'action d'un réactif azoté de formule générale $NH_2 - Z_3$ (dans laquelle $Z_3$ est défini comme précédemment) pour obtenir le composé de formule générale I désiré ;

$$Z_1 - Y - Z_2 - NHZ_3$$

que l'on peut, si désiré, salifier par addition d'une base alcaline lorsque $Z_3$ est un hydroxyle ou par addition d'un acide minéral ou organique lorsque $Z_3$ est un radical aminé ($NHR_1$) substitué ou non substitué.

De la même façon, les composés de formule générale II sont préparés par acylation de l'arginine par action d'un dérivé fonctionnel d'un acide organique carboxylique dans un solvant polaire en présence d'une base tertiaire puis estérification de la N-acylguanidine par un sulfate d'alcoyle en présence d'un alcanol, séparation de l'alcoylsulfate de N-acylguanidinate d'alcoyle, conversion en base et aminolyse de celle-ci par action d'un réactif de formule ${}_2HN - Z$ dans laquelle $Z_3$ est défini comme précédemment, pour former un composé de formule générale II

$$X_1 - \text{arginyl NH} - Z_3 \qquad \text{(II)}$$

dans laquelle X et $Z_3$ ont les définitions fournies précédemment.

Les composés de formule générale I ou de formule générale II constituent des réactifs précieux pour l'industrie biologique. Ils permettent le dosage des paramètres sanguins par voie enzymatique dans des conditions de sensibilité et de reproductivité jusqu'ici non encore atteintes.

Ils présentent le gros avantage par rapport aux réactifs du commerce d'être très solubles dans l'eau, de mettre en liberté des produits d'hydrolyse également très solubles et de ne pas nécessiter l'adjonction d'un tiers solvant.

Il est ainsi possible de doser les facteurs sanguins, les facteurs de la coagulation ou les facteurs enzymatiques impliqués dans la coagulation ou contenus dans les leucocytes à des concentrations de l'ordre de 0,01 partie par million (10 ppb). Le seuil de détection est donc très sensible et la mesure de l'activité enzymatique parfaitement linéaire.

En outre, l'extrême sensibilité de la mesure permet d'opérer sur des volumes très faibles et sur des solutions très diluées.

Le dosage du dérivé aminé $_2HN\text{-}Z_3$ mis en liberté par la réaction enzymatique se fait par une mesure électrochimique simple par rapport à une courbe d'étalonnage. La concentration optimale de réactif est de l'ordre de $5 \cdot 10^{-6}$ dans le milieu sanguin réactionnel.

L'invention comprend donc aussi l'emploi des composés de formule générale (I) ou de formule générale (II) comme réactifs biologiques et notamment pour le dosage ou la mise en évidence des différents facteurs sanguins.

C'est ainsi que les D-prolyl-L-phénylalanyl-L-arginyl-hydrazides permettent de doser la prekallicreine plasmatique, les D-valyl-L-leucyl-L-lysyl-hydrazides, permettent de déterminer la plasmine, les D-phénylalanyl-L-pipécolyl-L-arginyl-hydrazides permettent de doser la prothrombine, les N-benzoyl-L-isoleucyl-L-glutamyl-glycyl-L-arginyl-hydrazides permettent de doser le facteur X, les N-carbométhoxypropionyl-L-arginyl-L-prolyl-L-tyrosyl-hydrazides, l'α-chymotrypsine et les L-pyroglutamyl-L-prolyl-L-valyl-hydrazides permettent de doser l'élastase granulocytaire et les N-acylarginyl-hydrazides ou hydroxamates permettent de doser les protéases.

Les exemples suivants illustrent l'invention. Ils ne la limitent en aucune façon.

Exemple 1 : D-valyl-L-leucyl-L-lysyl-hydrazide.

Stade A
N-carboxyanhydride de la L-leucine.

On met en suspension 6,56 g de L-Leucine dans 250 ml de dioxane. On fait barboter à température ambiante très progressivement du phosgène jusqu'à saturation. Le ballon tricol dans lequel s'effectue la réaction est pourvu d'un réfrigérant à reflux permettant de piéger à basse température le phosgène en excès.

La suspension s'éclaircit rapidement et permet d'obtenir après 30 minutes de réaction une solution limpide, incolore. On laisse en contact pendant 1 heure puis on dégaze cette solution avant de la concentrer sous vide.

Le N carboxyanhydride de la L-leucine cristallise en fin de concentration.

Stade B
L-leucyl-L-lysine (ε Tosyl)

On met le dérivé ε tosylé de la lysine en solution à 5 % dans une solution aqueuse molaire de carbonate de sodium. Cette solution est refroidie à 0 °C, placée sous agitation très vive et l'on ajoute la quantité stœchiométrique de N-carboxyanhydride de la L-leucine en une seule fois. Le pH est maintenu entre 10 et 10,5 par de la soude 2N pendant 2 minutes, correspondant au temps nécessaire pour la dissolution complète du N-carboxyanhydride de la L-leucine.

Puis cette solution est acidifiée rapidement à pH 3,5 par de l'acide chlorhydrique concentré. Le rendement du couplage est de 99 %.

Stade C
D-valyl-L-leucyl-L-lysine (ε Tosyl)

La solution précédente contenant le L-leucyl-L-lysine (ε tosyl) est alcalinisée jusqu'à pH 10,5 par dissolution de carbonate de sodium puis refroidie à 0 °C et placée sous agitation très vive.

Une quantité stœchiométrique de N-carboxyanhydride de la D-valine (préparée selon le même mode opératoire que pour la L-leucine) est ajoutée en une seule fois en maintenant le pH entre 10 et 10,5 par de la soude 2N pendant 2 minutes.

Puis la solution est acidifiée par de l'acide chlorhydrique concentré jusqu'à pH 3,5 et dégazée sous vide avant d'être percolée sur résine sulfonique.

Le peptide fixé sur la résine est ensuite élué par une solution normale d'ammoniaque. L'éluat est concentré sous vide et le tripeptide D-valyl-L-leucyl-L-lysine (ε tosyl) cristallise en fin de concentration.

Stade D
D-valyl-L-leucyl-L-lysine

Le peptide ε tosylé sur la lysine est mis en suspension dans 250 ml de diméthoxy-1,2-éthane contenant en solution 12,8 g de naphtalène et 2,2 g de sodium.

L'agitation est maintenue pendant 4 heures à l'abri de l'humidité à température ambiante. 20 ml d'eau sont ensuite ajoutés goutte à goutte provoquant la décoloration de la solution. Le milieu réactionnel est ensuite placé dans une ampoule à décanter et la phase organique est extraite 5 fois par 100 ml d'eau. Les extraits aqueux sont percolés sur résine sulfonique puis élus par une solution normale d'ammoniaque.

L'éluat est concentré sous vide et le peptide D-valyl-L-leucyl-L-lysine ainsi obtenu est séché sous la forme d'une meringue.

Stade E
D-valyl-L-leucyl-L-lysinate de méthyle

La D-valyl-L-leucyl-L-lysine obtenue au stade D est redissoute dans 40 ml de méthanol. On ajoute ensuite 12 ml de sulfate de méthyle et on porte le mélange réactionnel au reflux pendant 90 minutes. On chasse le méthanol en excès. Il apparaît progressivement un précipité de (méthylsulfate). On laisse reposer à température ordinaire pendant une heure puis on sépare le précipité que l'on essore puis rince avec du méthanol et que l'on sèche sous vide.

Le méthylsulfate est remis en suspension dans le méthanol à 50 % puis on neutralise par addition d'une solution de bicarbonate de sodium à 10 %. Le D-valyl-L-leucyl-L-lysinate de méthyle est extrait au trichloréthane. On sépare la phase organique, la filtre, l'essore, la lave à l'eau, la sèche et l'évapore à sec. On redissout ensuite à chaud dans l'acétone d'où il précipite par refroidissement.

Après séparation puis essorage, on obtient le D-valyl-L-leucyl-L-lysinate de méthyle pur.

Stade F
D-valyl-L-leucyl-L-lysyl-hydrazide

On met en solution dans 125 ml de méthanol 4,1 g de D-valyl-L-leucyl-L-lysinate de méthyle obtenu au stade E puis on ajoute 10 ml d'une solution d'hydrate d'hydrazine. On porte le mélange à 45 °C, sous agitation en atmosphère inerte pendant 30 minutes. On laisse refroidir à température ordinaire puis on percole sur résine sulfonique.

Le peptide est ensuite élué par une solution normale d'ammoniaque. L'éluat est concentré à sec sous vide. Le résidu est ensuite neutralisé par de l'acide chlorhydrique concentré, puis cristallisé dans l'acétone. On le sépare par filtration, l'essore, le lave à l'acétone et le sèche sous vide. On obtient ainsi 2,6 g de D-valyl-L-leucyl-L-lysyl-hydrazide.

Exemple 2 : D-phénylalanyl-L-pipécolyl-L-arginyl-hydrazide.

Stade A
L-pipécolyl-L-arginine.

On prépare le N-carboxyanhydride de l'acide pipécolique selon le mode opératoire de l'exemple 1, stade A, puis on l'ajoute à une solution aqueuse d'arginine et de carbonate de sodium molaire en maintenant le pH entre 10 et 10,5, par addition de soude 2N. On maintient à 0 °C par un bain réfrigérant extérieur sous forte agitation. Au bout de 2 minutes, tout en maintenant la température à 0 °C, on amène le pH à 3,5 par addition d'acide chlorhydrique concentré. Le peptide formé est séparé par passage de la solution sur colonne de résine carboxylique puis élué par une solution d'ammoniaque normale. On concentre l'éluat à sec pour obtenir le dipeptide pur.

Stade B
N-carboxyanhydride de la D-phénylalanine

En opérant comme au stade A de l'exemple 1, au départ de la phénylalanine et du phosgène, on forme le N-carboxyanhydride de D-phénylalanine que l'on utilise tel quel.

Stade C
D-phénylalanyl-L-pipécolyl-L-arginine.

En opérant comme au stade B de l'exemple 1, au départ du N-carboxyanhydride de la D-phénylalanine du L-pipécolyl-L-arginine, on obtient après chromatographie sur résine carboxylique, la D-phénylalanyl-L-pipécolyl-L-arginine.

Stade D
D-phénylalanyl-L-pipécolyl-L-argininate d'éthyle.

On dissout la D-phénylalanyl-L-pipécolyl-L-arginine dans 50 ml d'éthanol. On y ajoute 4,5 ml de sulfate diéthylique et on porte le mélange au reflux de l'éthanol pendant 2 heures. La solution obtenue est ensuite concentrée et il apparaît un précipité d'éthylsulfate de D-phénylalanyl-L-pipécolyl-L-argininate d'éthyle que l'on sépare par filtration, essore puis lave à l'acétone et sèche. On le dissout dans une solution molaire de bicarbonate de sodium et l'ester base est extrait par du chlorure de méthylène. On sépare la phase chlorométhylénique que l'on lave trois fois à l'eau puis sèche sur sulfate de sodium, décolore au charbon, filtre et évapore à sec sous vide. On recueille ainsi le D-phénylalanyl-L-pipécolyl-L-argininate d'éthyle sous forme d'un produit huileux insoluble dans l'eau et soluble dans les solvants organiques.

Stade E
D-phénylalanyl-L-pipécolyl-L-arginyl-hydrazide.

On met en solution le D-phénylalanyl-L-pipécolyl-L-argininate d'éthyle dans 25 ml d'éthanol. On ajoute progressivement 10 ml d'hydrate d'hydrazine et on porte le mélange à 50 °C pendant 1 heure. On laisse ensuite refroidir et percole sur une résine carboxylique.

L'élution par une solution d'ammoniaque normale puis la concentration à sec de cet éluat permet d'obtenir une huile qui cristallise dans un mélange méthanol-acétone. On sépare le précipité d'hydrazide que l'on essore à fond, lave à l'acétone puis sèche.

Exemple 3 : Acide N-formylarginyl-hydroxamique

Stade A
N-formylargininate de méthyle

En opérant comme à l'exemple 1, stade E, au départ de la N-formylarginine, du méthanol et du sulfate de méthyle, on obtient le N-formylargininate de méthyle sous forme d'une huile incolore.

Stade B
Acide N-formylarginyl-hydroxamique

On dissout le N-formylargininate de méthyle dans 25 ml de méthanol puis on ajoute 7 ml d'une solution de chlorhydrate d'hydroxylamine à 10 % dans l'eau, puis 2,5 g de carbonate de potassium. On porte le mélange au reflux pendant une heure, laisse refroidir à température ordinaire puis percole sur résine carboxylique. Le filtrat concentré sous vide est cristallisé en fin de concentration. On filtre le précipité, on l'essore, le rince avec un peu d'acétone, puis le sèche sous vide. On obtient ainsi l'acide N-formylarginyl-hydroxamique.

Exemple 4 : (N-benzoylarginyl) N'-méthyl-hydrazine

Stade A
N-benzoylargininate de méthyle

On dissout 4,5 g de N-benzoylarginine dans 35 ml de méthanol puis on ajoute 2,5 ml de sulfate de méthyle. On maintient sous agitation pendant 2 heures à 60 °C puis on sépare le précipité de méthylsulfate.

Celui-ci est converti en N-benzoylargininate de méthyl par alcalinisation avec une solution de bicarbonate de sodium.

Stade B
(N-benzoylarginyl) (N'-méthylhydrazide)

Le N-benzoylargininate de méthyle est mis en solution dans 25 ml de méthanol puis on ajoute 2,5 ml d'une solution de méthylhydrazine à 20 % dans le méthanol. On maintient sous forte agitation à 60 °C, pendant deux heures puis on laisse reposer la suspension cristalline pendant une nuit. On sépare les cristaux par filtration, les essore, les lave au méthanol et les sèche sous vide. On les remet en suspension dans le méthanol puis fait barboter un courant de gaz chlorhydrique pendant 30 minutes. On laisse décanter le précipité de chlorhydrate qui s'est formé ; on le filtre, l'essore, le rince avec un peu de méthanol et le sèche sous vide. Le méthylhydrazide est ainsi obtenu pur sous forme de chlorhydrate. Le chlorhydrate peut être transformé en hydrazide par passage en milieu alcalin par addition de soude 2N.

**Revendications**

1. Méthode de dosage d'enzymes, tels que dosages de facteurs sanguins, de facteurs de coagulation, de facteurs enzymatiques, caractérisée :
en ce que ladite méthode met en œuvre un réactif de dosage répondant à la formule

$$Z_1 - Y(Z_2)\,NHZ_3\,, \qquad (I)$$

et dans laquelle :

$Z_1$ représente un reste d'amino-acide choisi parmi les radicaux D-valyl, D-propyl, D-phénylalanyl, L-isoleucyl, L-glutamyl, L-pyroglutamyl, (N-benzoyl-L-isoleucyl-L-glytamyl) (N carbométhoxy-propionyl-L-arginyl) et autres amino-acides et peptides,

Y est un reste d'amino-acide choisi dans le groupe constitué par un radical L-leucyl, L-phénylalanyl, L-pipécolyl, glycyl et L-propyl et autres amino-acides.

$Z_2$ est un reste d'amino-acide choisi dans le groupe constitué par un radical L-lysyl, L-arginyl, L-tyrosyl et L-valyl et autres amino-acides,

$Z_3$ représente un radical de formule $NH_2$, $NHR_1$ ou OH (dans laquelle $R_1$ est un radical alcoyle inférieur, un radical alcoyle inférieur substitué, un radical aryle ou un radical aryle substitué), en ce qu'elle consiste à doser les dérivés

$$H_2N-Z_3$$

libéré par réaction enzymatique ;

2. Méthode selon la revendication 1, caractérisée en ce que ledit réactif répond plus particulièrement à la formule

$$Z_1 - Y - (L - arginyl)\,NH - Z_3 \qquad (I_A)$$

dans laquelle :

$Z'_1$ est un reste d'acide aminé choisi dans le groupe constitué par le radical D-propyl, D-valyl, D-phénylalanyl, N-benzoyl-L-isoleucyl-L-glutamyl, D-isoleucyl et L-glutamyl et autres amino-acides,

Y' est un reste d'amino-acide choisi dans le groupe constitué par un radical L-phénylalanyl, L-leucyl, L-pipécolyl, glycyl et L-propyl et autres amino-acides,

$Z_3$ est défini comme précédemment ;

3. Méthode selon la revendication 1, caractérisée en ce que ledit réactif répond plus particulièrement à la formule

$$Z_1 - Y - Z_2\,NHOH \qquad (I_B)$$

dans laquelle les radicaux $Z_1$, $Z_2$ et Y sont définis comme précédemment ;

4. Méthode selon la revendication 1, caractérisée en ce que ledit réactif répond plus particulièrement à la formule

$$Z_1 - Y - Z_2\,NHNH_2 \qquad (I_C)$$

dans laquelle les radicaux $Z_1$, $Z_2$ et Y sont définis comme précédemment ;

5. Méthode selon la revendication 1, caractérisée en ce que ledit réactif répond plus particulièrement à la formule

$$Z_1 - Y - Z_2\,NH - NHR_2 \qquad (I_D)$$

dans laquelle les radicaux $Z_1$, $Z_2$ et Y sont définis comme précédemment,

6. Méthode selon la revendication 5, caractérisée en ce que ledit réactif répond plus particulièrement à la formule

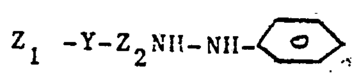

dans laquelle les radicaux $Z_1$, $Z_2$ sont définis comme précédemment ;

7. Méthode selon la revendication 1, caractérisée en ce que dans la formule générale (I) dudit réactif :

Y est un acide aminé,

$Z_1$ est un acide aminé pouvant comporter un groupement de l'ensemble de groupements comprenant le groupement aminoacide libre et le groupement amino-acide acylé, ce dernier radical acyle pouvant être soit un résidu d'un acide organique carboxylique, aliphatique ou aromatique, comportant de 1 à 10 atomes de carbone, soit un groupement peptidique, et

$Z_2$ est un radical d'amino-acide ;

8. Méthode selon l'une quelconque des revendications précédentes, caractérisée en ce que le dosage du dérivé $H_2N-Z_3$ est réalisé par mesure électrochimique par rapport à une courbe d'étalonnage ;

9. Méthode selon la revendication 8, caractérisée en ce qu'appliquée au dosage sanguin, la concentration du réactif est de l'ordre de $10^{-6}$ mole/l.

**Claims**

1. Method for determining quantitatively enzymes, such as determining blood factors, coagulation

factors, and enzyme factors characterized by the fact that said method applies a reagent corresponding to the formula

$$Z_1 - Y - (Z_2)\,NHZ_3 \qquad (I)$$

and in which

$Z_1$ is an amino acid residue selected from the radicals D-valyl, D-propyl, D-phenylalanyl, L-isoleucyl, L-glutamyl, L-pyroglutamyl, (N-benzoyl-L-isoleucyl-L-glutamyl) (N carbomethoxypropionyl-L-arginyl) and other amino acids and peptides.

Y is an amino acid residue selected from the group consisting of a radical L-leucyl, L-phenylalanyl, L-pipecolyl, glycyl, L-propyl and other amino acids.

$Z_2$ is an amino acid residue selected from the group consisting of a radical L-lysyl, L-arginyl, L-tyrosyl and L-valyl and other amino acids.

$Z_3$ is a radical, the formula of which is $NH_2$, $NHR_1$, or OH (in which $R_1$ is a lower alkyl radical, a substituted lower alkyl radical, an aryl radical or a substituted aryl radical), and in that it consists in determining the derivatives

$$H_2N - Z_3$$

released by enzymatic reaction ;

2. Method in accordance with claim 1, characterized by the fact that said reagent corresponds more particularly to the formula

$$Z_1 - Y - (L - arginyl)\,NH - Z_3 \qquad (I_A)$$

in which

$Z'_1$ is an amino acid residue selected from the group consisting of the radicals D-propyl, D-valyl, D-phenylalanyl, N-benzoyl-L-isoleucyl-L-glutamyl, D-isoleucyl, L-glutamyl and other amino acids,

Y' is an amino acid residue selected from the group consisting of a radical L-phenylalanyl, L-leucyl, L-pipecolyl, glycyl, L-propyl and other amino acids,

$Z_3$ is defined as before ;

3. Method in accordance with claim 1, characterized by the fact that said reagent corresponds more particularly to the formula

$$Z_1 - Y - Z_2\,NHOH \qquad (I_B)$$

in which the radicals $Z_1$, $Z_2$ and Y are defined as before ;

4. Method in accordance with claim 1, characterized by the fact that said reagent corresponds more particularly to the formula

$$Z_1 - Y - Z_2\,NHNH_2 \qquad (I_C)$$

in which the radicals $Z_1$, $Z_2$ and Y are defined as before ;

5. Method in accordance with claim 1, characterized by the fact that said reagent corresponds more particularly to the formula

$$Z_1 - Y - Z_2\,NH - NHR_2 \qquad (I_D)$$

in which the radicals $Z_1$, $Z_2$ and Y are defined as before ;

6. Method in accordance with claim 5, characterized by the fact that said reagent corresponds more particularly to the formula

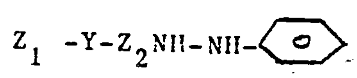

in which the radicals $Z_1$, $Z_2$ are defined as before ;

7. Method in accordance with claim 1, characterized by the fact that in the general formula (I) of said reagent :

Y is an amino acid,

$Z_1$ is an amino acid which may comprise a group from the overall grouping including free amino acid groups and acylated amino acid groups, the latter acyl radical can be either an organic carboxylic, aliphatic or aromatic acid residue containing 1 to 10 carbon atoms or a peptide group ; and

$Z_2$ is an amino acid radical ;

8. Method in accordance with any of the foregoing claims, characterized by the fact that the

quantitative determining of the $H_2N$-$Z_3$ derivative is done by electrochemical measurement in relation to a standardization curve.

9. Method in accordance with claim 8, characterized by the fact that the concentration of the reagent applied to the blood determining is of the order of $10^{-6}$ mol/liter.

**Patentansprüche**

1. Verfahren zum Dosieren von Enzymen, wie dem Dosieren von Blutwerten, Coagulationswerten, enzymatischen Werten, dadurch gekennzeichnet,
daß das Verfahren ein Dosierreagens verarbeitet, nach der Formel

$$Z_1 - Y(Z_2)\,NHZ_3\,, \qquad (I)$$

mit den folgenden Bedeutungen :

$Z_1$ stellt einen Aminosäurenrest dar, und zwar aus den radikalen D-Valyl, D-Prolyl, D-Phenylalanyl, L-Isoleucyl, L-Glutamyl) L-Pyroglutamyl (N-Benzoyl-L-Isoleucyl-L-glytamyl) (N Carbomethoxy-Propionyl-L-Arginyl) sowie anderen Aminosäuren und Peptiden,

Y ist ein Aminosäurenrest aus der Gruppe, die aus einem Radikal L-Leucyl, L-Phenylalanyl, L-Pipecolyl, Glycyl sowie L-Propyl und anderen Aminosäuren besteht,

$Z_2$ ist ein Aminosäurenrest, ausgewählt aus der Gruppe, die aus einem Radikal L-Lysyl, L-Arginyl, L-Tyrosyl sowie L-Valyl und anderen Aminosäuren besteht,

$Z_3$ ist ein Radikal der Formel $NH_2$, $NHR_1$ oder OH (wobei $R_1$ ein niederes Alkylradikal ist, ein substituiertes, niederes Alkylradikal, ein Arylradikal oder ein substituiertes Arylradikal), und daß die durch die enzymatische Reaktion freigesetzten Derivate

$$H_2N - Z_3$$

dosiert werden.

2. Verfahren nach Anspruch 1, dadurch gekennzeichnet, daß das genannte Reagens insbesondere der Formel gehorcht

$$Z'_1 - Y' - (L - Arginyl)\,NH - Z_3 \qquad (I_A)$$

mit den folgenden Bedeutungen :

$Z'_1$ ist ein aminierter Säurerest, ausgewählt aus der Gruppe, die aus dem Radikal D-Propyl, D-Valyl, D-Phenylalanyl, N-Benzoyl-L-Isoleucyl-L-Glutamyl, D-Isoleucyl sowie L-Glutamyl und anderen Aminosäuren besteht,

Y' ist ein Aminosäurenrest aus der Gruppe, die aus einem Radikal L-Phenylalanyl, L-Leucyl, L-Pipecolyl, Glycyl sowie L-Propyl und anderen Aminosäuren besteht,

$Z_3$ ist wie vorausgehend definiert.

3. Verfahren nach Anspruch 1, dadurch gekennzeichnet, daß das genannte Reagens insbesondere der folgenden Formel gehorcht :

$$Z_1 - Y - Z_2\,NHOH \qquad (I_B)$$

wobei die Radikale $Z_1$, $Z_2$ und Y wie vorausgehend definiert sind.

4. Verfahren nach Anspruch 1, dadurch gekennzeichnet, daß das genannte Reagens insbesondere der Formel gehorcht

$$Z_1 - Y - Z_2\,NHNH_2 \qquad (I_C)$$

wobei die Radikale $Z_1$, $Z_2$ und Y wie vorausgehend definiert sind.

5. Verfahren nach Anspruch 1, dadurch gekennzeichnet, daß das genannte Reagens insbesondere der Formel gehorcht

$$Z_1 - Y - Z_2\,NH - NHR_2 \qquad (I_D)$$

wobei die Radikale $Z_1$, $Z_2$ und Y wie vorausgehend definiert sind.

6. Verfahren nach Anspruch 5, dadurch gekennzeichnet, daß das genannte Reagens insbesondere der Formel gehorcht

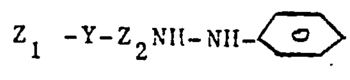

wobei die Radikale $Z_1$, $Z_2$ wie vorausgegangen definiert sind.

7. Verfahren nach Anspruch 1, dadurch gekennzeichnet, daß in der allgemeinen Formel (I) des genannten Reagens

Y eine aminierte Säure ist,

$Z_1$ eine aminierte Säure ist, die eine Gruppe der Gesamtheit der Gruppen aufweist, enthaltend die Gruppe der freien Aminosäuren und die Gruppe der acilierten Aminosäuren, wobei das letztgenannte Acylradikal der Rest einer organischen carboxylischen, aliphatischen oder aromatischen Säure sein kann, enthaltend 1-10 Kohlenstoffatome, oder eine peptidische Gruppe, und

$Z_2$ ein Aminosäurenradikal ist.

8. Verfahren nach einem der Ansprüche 1-7, dadurch gekennzeichnet, daß die Dosierung des Derivates $H_2N\text{-}Z_3$ verwirklicht wird durch elektrochemisches Messen in Bezug auf eine Eichkurve.

9. Verfahren nach Anspruch 8, dadurch gekennzeichnet, daß die Konzentration des Reagens bei der Blutdosierung angewandt in der Größenordnung von $10^{-6}$ mol/l liegt.